# EUROPEAN PATENT SPECIFICATION

(11) **EP 2 754 403 B1**
(45) Date of publication and mention of the grant of the patent: **13.06.2018**
(21) Application number: 14150755.8
(22) Date of filing: 10.01.2014
(51) Int. Cl.: A61B 18/14, A61B 18/00

(54) **Surgical forceps**
Chirurgische Zange
Pince chirurgicale

(30) Priority: 10.01.2013 US 201361751121 P; 28.10.2013 US 201314064310
(43) Date of publication of application: 16.07.2014
(73) Proprietor: Covidien LP, Mansfield, MA 02048 (US)
(72) Inventor: Reschke, Arlen J., Longmont, CO 80501 (US); Dickhans, William J., Longmont, CO 80503 (US); Couture, Gary M., Longmont, CO 80501 (US); Mueller, Peter M., Frederick, CO 80516 (US); Coulson, Rebecca J., Lyons, CO 80540 (US); Anderson, Sara E., Erie, CO 80516 (US)
(74) Representative: Soames, Candida Jane

(56) References cited:
- US-A1- 2001 037 109
- US-A1- 2008 195 093
- US-A1- 2012 289 957
- US-B1- 6 273 887
- US-B2- 7 270 660

## Description

### BACKGROUND

### Technical Field

The present disclosure relates to a surgical devices and, more particularly, to surgical forceps for grasping, treating, and/or cutting tissue.

### Background of Related Art

A forceps is a plier-like instrument which relies on mechanical action between its jaws to grasp, clamp and constrict vessels or tissue. Electrosurgical forceps utilize both mechanical clamping action and electrical energy to affect hemostasis by heating tissue and blood vessels to coagulate and/or cauterize tissue. Certain surgical procedures require more than simply cauterizing tissue and rely on the unique combination of clamping pressure, precise electrosurgical energy control and gap distance (i.e., distance between opposing jaw members when closed about tissue) to "seal" tissue, vessels and certain vascular bundles. Typically, once a vessel is sealed, the surgeon has to accurately sever the vessel along the newly formed tissue seal. Accordingly, many vessel sealing instruments have been designed which incorporate a knife or blade member which effectively severs the tissue after forming a tissue seal. Alternatively or additionally, energy-based tissue division may be effected.

US 2008/195093 A1 discloses an end effector assembly including a pair of opposing first and second jaw members, each jaw member including an electrically conductive tissue contacting surface and at least one of the jaw members including an electrically conductive cutting element. However, US 2008/195093 does not disclose a first insulative member configured to guide the cutting electrode into alignment with the first insulative member.

US 2001/0037109 A1 discloses a treatment tool for coagulating and incising tissue, comprising a pair of jaws.

### SUMMARY

As used herein, the term "distal" refers to the portion that is being described which is further from a user, while the term "proximal" refers to the portion that is being described which is closer to a user. Further, to the extent consistent, any of the aspects described herein may be used in conjunction with any or all of the other aspects described herein.

According to the present invention, there is provided forceps, comprising: an end effector assembly including first and second jaw members, at least one of the jaw members movable relative to the other between a spaced-apart position and an approximated position for grasping tissue therebetween, each jaw member including an electrically-conductive tissue-contacting surface adapted to connect to a source of energy to treat tissue grasped between the jaw members, the first jaw member including a cutting electrode adapted to connect to the source of energy to cut tissue grasped between the jaw members, the second jaw member including a first insulative member positioned to oppose the cutting electrode, the first insulative member configured to guide the cutting electrode into alignment with the first insulative member upon approximation of the jaw members to thereby align the jaw members relative to one another upon approximation of the jaw members, wherein the first insulative member defines a cut-out formed from at least one angled surface, the at least one angled surface configured to guide the cutting electrode into alignment within the cut-out upon approximation of the jaw members.

In embodiments, the cut-out is defined by a base surface of the first insulative member and a pair of angled surfaces of the first insulative member disposed on either side of the base surface. The angled surfaces are configured to guide the cutting electrode into alignment with the base surface.

In embodiments, the end effector assembly of the forceps further includes a second insulative member surrounding the cutting electrode and configured to electrically insulate the cutting electrode and tissue-contacting surface of the first jaw member from one another.

The cutting electrode contacts the first insulative member to define a minimum gap distance between the first and second jaw members.

The tissue-contacting surfaces of the jaw members are configured to conduct energy therebetween and through tissue grasped between the jaw members to treat tissue.

The cutting electrode is configured to conduct energy to one or both of the tissue-contacting surfaces and through tissue grasped between the jaw members to cut tissue.

The electrically conductive tissue cutting surface of the second jaw member includes a longitudinal slot extending therethrough and the fist insulative member is disposed therein.

In embodiments, alignment of the jaw members is for maintaining substantially equal spacing between the cutting electrode and the tissue contacting surface of the second jaw member on both sides of the cutting electrode and/or the longitudinal slot.

In embodiments, the tissue-contacting surfaces of the jaw members are configured to conduct energy therebetween and through tissue grasped between the jaw members to treat tissue.

In embodiments, the cutting electrode is configured to conduct energy to one or both of the tissue-contacting surfaces and through tissue grasped between the jaw members to cut tissue. The invention is defined in claim 1. Further aspects and preferred embodiments are defined in the dependent claims. Aspects, embodiments and examples of the present disclosure which do not fall under the scope of the appended claims are merely provided for illustrative purposes.

### BRIEF DESCRIPTION OF THE DRAWINGS

Various aspects and features of the present disclosure are described herein with reference to the drawings wherein:
FIG. 1 is a front, side, perspective view of an endoscopic surgical forceps configured for use in accordance with the present disclosure;
FIG. 2 is a front, side, perspective view of an open surgical forceps configured for use in accordance with the present disclosure;
FIG. 3A is a front, side, perspective view of an end effector assembly configured for use with the forceps of FIGS. 1 or 2;
FIG. 3B is a front, side, perspective view of another end effector assembly configured for use with the forceps of FIGS. 1 or 2;
FIG. 4 is a transverse, cross-sectional view of the end effector assembly of FIG. 3B;
FIGS. 5A-5D are top views of various different configurations of jaw members configured for use with the end effector assembly of FIG. 3B;
FIG. 6A is a front, side, perspective view of another end effector assembly configured for use with the forceps of FIGS. 1 or 2;
FIG. 6B is a front view of the proximal flanges of the jaw members of the end effector assembly of FIG. 6A;
FIG. 7A is an exploded, front, side, perspective view of another end effector assembly configured for use with the forceps of FIGS. 1 or 2; and
FIG. 7B is a side view of the replaceable components of the end effector assembly of FIG. 7A.

### DETAILED DESCRIPTION

There is described a forceps including an end effector assembly having first and second jaw members movable between a spaced-apart position and an approximated position for grasping tissue therebetween. Each jaw member includes an electrically-conductive tissue-contacting surface adapted to connect to a source of energy to treat tissue grasped between the jaw members. The first jaw member includes a cutting electrode adapted to connect to the source of energy to cut tissue grasped between the jaw members. The second jaw member includes an insulative member positioned to oppose the cutting electrode. The insulative member defines a non-uniform configuration, or non-uniform shape in a plan view of the tissue contacting surface of the second jaw member, along a length thereof to facilitate cutting of tissue.

In embodiments, the insulative member increases in width from a proximal end to a distal end thereof.

In embodiments, the insulative member includes an expanded distal portion. A distal end of the cutting electrode may be configured for positioning adjacent the expanded distal portion of the insulative member upon movement of the jaw members to the approximated position.

In embodiments, the insulative member defines a proximal portion, a distal portion, and a central portion interdisposed between the proximal and distal portions. A part of (or the entire) central portion defines a reduced width relative to the proximal and distal portions.

In embodiments, the insulative member defines an irregular outer peripheral edge. In particular, the insulative member may define a zigzagged outer peripheral edge.

In embodiments, the tissue contacting surface of the second jaw member includes a longitudinal slot defined therethrough and the insulative member is disposed therein, wherein the insulative member and the longitudinal slot defines the non-uniform configuration.

In embodiments, the insulative member includes one or more relatively laterally narrow portions and one or more relatively laterally expanded portions along the length thereof.

In embodiments, the expanded portion or portions provide for increased lateral spacing between the cutting electrode and the tissue contact surface of the second jaw member on both sides of the insulative member.

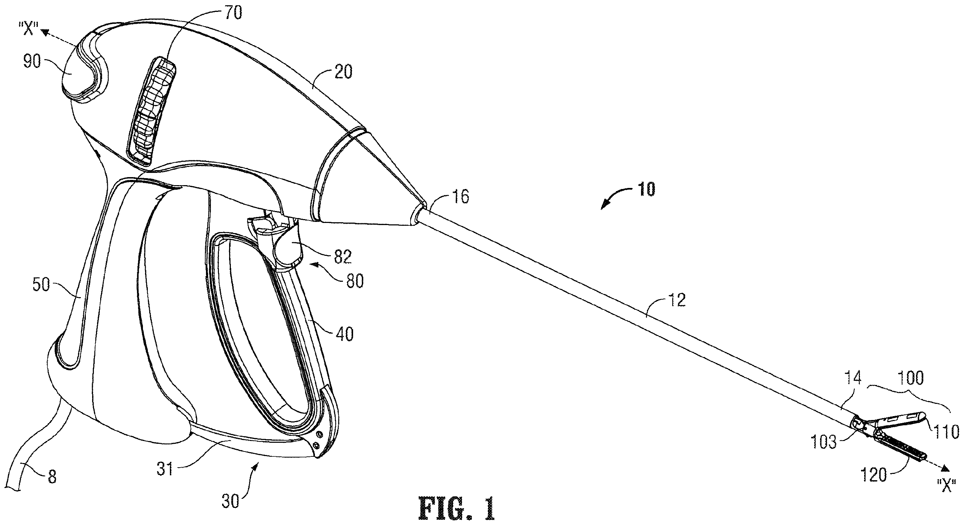

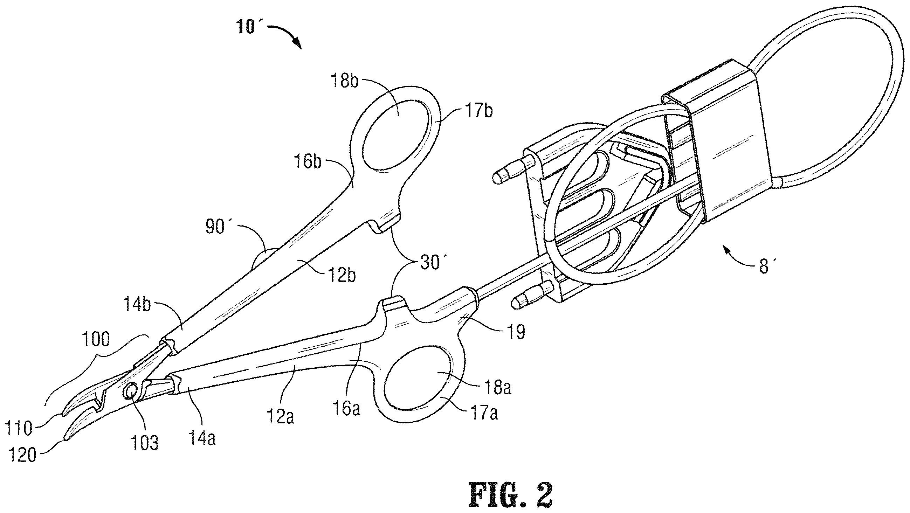

Referring now to FIGS. 1 and 2, FIG. 1 depicts a forceps 10 for use in connection with endoscopic surgical procedures and FIG. 2 depicts an open forceps 10' contemplated for use in connection with traditional open surgical procedures. For the purposes herein, either an endoscopic device, e.g., forceps 10, an open device, e.g., forceps 10', or any other suitable surgical device may be utilized in accordance with the present disclosure. Obviously, different electrical and mechanical connections and considerations apply to each particular type of device, however, the aspects and features of the present disclosure remain generally consistent regardless of the particular device used.

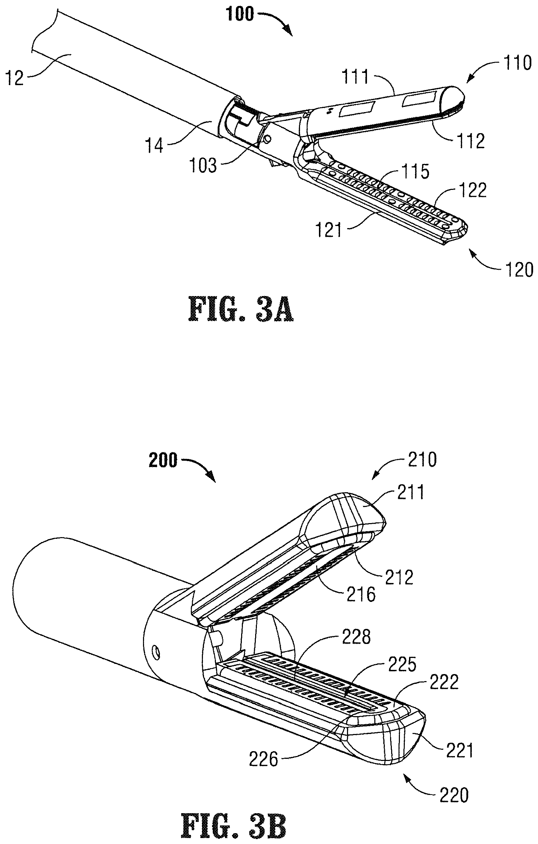

Turning now to FIG. 1, an endoscopic forceps 10 is provided defining a longitudinal axis "X-X" and including a housing 20, a handle assembly 30, a rotating assembly 70, a trigger assembly 80 and an end effector assembly 100. Forceps 10 further includes a shaft 12 having a distal end 14 configured to mechanically engage end effector assembly 100 and a proximal end 16 that mechanically engages housing 20. Forceps 10 also includes cable 8 that connects forceps 10 to an energy source (not shown), e.g., a generator or other suitable power source, although forceps 10 may alternatively be configured as a battery-powered device. Cable 8 includes a wire (or wires) (not shown) extending therethrough that has sufficient length to extend through shaft 12 in order to provide energy to at least one of tissue-contacting surfaces 112, 122 (FIG. 3A) of jaw members 110, 120, respectively. An activation switch 90 is provided on housing 20 for selectively supplying energy to jaw members 110, 120.

With continued reference to FIG. 1, handle assembly 30 includes fixed handle 50 and a moveable handle 40. Fixed handle 50 is integrally associated with housing 20 and handle 40 is moveable relative to fixed handle 50. Rotating assembly 70 is rotatable in either direction about a longitudinal axis "X-X" to rotate end effector 100 about longitudinal axis "X-X." Housing 20 houses the internal working components of forceps 10.

Continuing with reference to FIG. 1, moveable handle 40 of handle assembly 30 is ultimately connected to a drive assembly (not shown) that, together, mechanically cooperate to impart movement of jaw members 110 and 120 between a spaced-apart position and an approximated position to grasp tissue disposed between jaw members 110, 120. As shown in FIG. 1, moveable handle 40 is initially spaced-apart from fixed handle 50 and, correspondingly, jaw members 110, 120 are in the spaced-apart position. Moveable handle 40 is depressible from this initial position to a depressed position corresponding to the approximated position of jaw members 110, 120. In some embodiments, a knife assembly (not shown) is provided. Trigger 82 of trigger assembly 80 is operably coupled to the knife assembly (not shown) for selectively translating a knife blade (not shown) through a knife channel 115 (FIG. 3A) defined within one or both of jaw members 110, 120 to cut tissue disposed between jaw members 110, 120 to cut tissue.

Referring now to FIG. 2, an open forceps 10' is shown including two elongated shafts 12a and 12b, each having a proximal end 16a and 16b, and a distal end 14a and 14b, respectively. Similar to forceps 10 (FIG. 1), forceps 10' is configured for use with end effector assembly 100. More specifically, end effector assembly 100 is attached to distal ends 14a and 14b of shafts 12a and 12b, respectively. As mentioned above, end effector assembly 100 includes a pair of opposing jaw members 110 and 120 that are pivotably connected about a pivot 103. Each shaft 12a and 12b includes a handle 17a and 17b disposed at the proximal end 16a and 16b thereof. Each handle 17a and 17b defines a finger hole 18a and 18b therethrough for receiving a finger of the user. As can be appreciated, finger holes 18a and 18b facilitate movement of the shafts 12a and 12b relative to one another that, in turn, pivots jaw members 110 and 120 from an open position, wherein the jaw members 110 and 120 are disposed in spaced-apart relation relative to one another, to a closed position, wherein the jaw members 110 and 120 cooperate to grasp tissue therebetween.

A ratchet assembly 30' may be included for selectively locking the jaw members 110 and 120 relative to one another at various positions during pivoting. Ratchet assembly 30' may include graduations or other visual markings that enable the user to easily and quickly ascertain and control the amount of closure force desired between the jaw members 110 and 120. Forceps 10 (FIG. 1) may also include a ratchet assembly 31 (FIG. 1) for similar purposes.

With continued reference to FIG. 2, one of the shafts, e.g., shaft 12a, includes a proximal shaft connector 19 which is designed to connect the forceps 10' to a source of energy (not shown), e.g., a generator. Proximal shaft connector 19 secures an electrosurgical cable 8' to forceps 10' such that the user may selectively apply energy to jaw members 110 and 120, as needed. One of the shafts, e.g., shaft 12a, includes an activation switch 90' for selectively supplying energy to jaw members 110, 120.

Referring to FIGS. 3A and 3B, end effector assemblies configured for use with forceps 10 (FIG. 1), forceps 10' (FIG. 2), or any other suitable surgical device are generally designated as end effector assemblies 100, 200, respectively. However, for purposes of simplicity, end effector assemblies 100, 200 will be described herein as configured for use with forceps 10 (FIG. 1). End effector assemblies 100, 200 are generally similar to one another except that end effector assembly 100 (FIG. 3A) is configured to permit translation of a knife blade (not shown) through knife slot(s) 115 defined within one or both of jaw members 110, 120 to dynamically cut tissue therebetween, while end effector assembly 200 (FIG. 3B) includes an electrical cutting assembly 225 configured to conduct energy through tissue to statically cut tissue grasped between jaw members 210, 220. Each of end effector assemblies 100, 200 will be described, in turn, below.

With reference to FIG. 3A, each of jaw members 110, 120 of end effector assembly 100 includes an outer insulative jaw housing 111, 121 and an electrically-conductive tissue-contacting surface 112, 122, respectively. Tissue-contacting surfaces 112, 122 are electrically coupled to activation switch 90 (FIG. 1) and the source of energy (not shown), e.g., via the wires (not shown) extending from cable 8 (FIG. 1) through forceps 10 (FIG. 1), such that energy may be selectively supplied to tissue-contacting surface 112 and/or tissue-contacting surface 122 and conducted therebetween and through tissue disposed between jaw members 110, 120 to treat, e.g., seal, tissue. End effector assembly 100 is designed as a unilateral assembly, i.e., where jaw member 120 is fixed relative to shaft 12 and jaw member 110 is moveable about pivot 103 relative to shaft 12 and fixed jaw member 120. However, end effector assembly 100 may alternatively be configured as a bilateral assembly, i.e., where both jaw member 110 and jaw member 120 are moveable about a pivot 103 relative to one another and to shaft 12. A knife channel 115 extends longitudinally through one (or both) jaw members 110, 120, e.g., jaw member 110, to facilitate reciprocation of a knife blade (not shown) between jaw members 110, 120 to cut tissue disposed therebetween, e.g., upon actuation of trigger 82 of trigger assembly 80 (see FIG. 1). The knife blade (not shown) translating though knife channel 115 and between jaw members 110, 120 may be configured for mechanical cutting, or may be energizable, e.g., electrically coupled to the source of energy (not shown) via one or more wires (not shown) of cable 8 (FIG. 1), for electromechanically cutting tissue.

Referring to FIG. 3B, similar to end effector assembly 100 (FIG. 3A), jaw members 210, 220 of end effector assembly 200 each include an outer insulative jaw housing 211, 221 and an electrically-conductive tissue-contacting surface 212, 222, respectively. Tissue-contacting surfaces 212, 222 are electrically coupled to activation switch 90 (FIG. 1) and the source of energy (not shown), e.g., via wires (not shown) extending from cable 8 (FIG. 1) through forceps 10 (FIG. 1), for selectively supplying energy to tissue-contacting surface 212 and/or tissue-contacting surface 222 to treat, e.g., seal, tissue, in a first mode of operation. End effector assembly 200 is designed as a unilateral assembly, although end effector assembly 200 may alternatively be configured as a bilateral assembly. One of the jaw members 210, 220 of end effector assembly 200, e.g., jaw member 220, includes an electrical cutting assembly 225 disposed within a longitudinal slot extending along tissue-contacting surface 222 and jaw member 220. Electrical cutting assembly 225 includes an insulating member 226 and a cutting electrode 228. Insulating member 226 is interdisposed between cutting electrode 228 and tissue-contacting surface 222 to electrically insulate cutting electrode 228 and tissue-contacting surface 222 from one another. Cutting electrode 228 is electrically coupled to activation switch 90 (FIG. 1) and the source of energy (not shown), e.g., via one or more wires (not shown), for selectively supplying energy to cutting electrode 228 for conduction through tissue and to either or both of tissue-contacting surfaces 212, 222 to electrically or electromechanically cut tissue in a second mode of operation. An insulating member 216 disposed within a longitudinal slot extending along tissue-contacting surface 212 of jaw member 210 is provided to oppose cutting electrode 228.

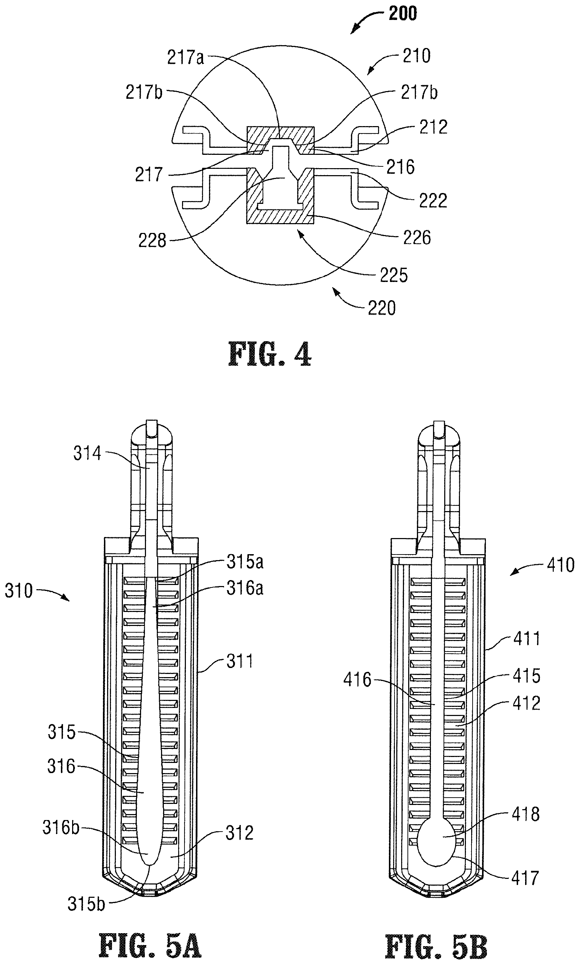

The various features and configurations described below with reference to FIGS. 5A-7B are configured for use with an end effector assembly, e.g., dynamic cutting end effector assembly 100 (FIG. 3A) and/or static cutting end effector assembly 200 (FIG. 3B), of a surgical forceps, e.g., endoscopic surgical forceps 10 (FIG. 1) and/or open surgical forceps 10' (FIG. 2), for facilitating effective tissue sealing and/or effective tissue cutting (dynamically and/or statically). To the extent consistent with one another, any of the features and configurations described hereinbelow may be used in conjunction with any or all of the other features and configurations described hereinbelow. Further, any of the features and configurations described hereinbelow may be incorporated into or used with any of end effector assemblies 100, 200 (FIGS. 3A, 3B, respectively), forceps 10, 10' (FIGS. 1, 2, respectively), or any other suitable surgical devices or components thereof.

Turning now to FIG. 4, as described above, end effector assembly 200 includes first and second jaw members 210, 220, each including an electrically-conductive tissue-contacting surface 212, 222, respectively, and a longitudinal slot extending therethrough. Jaw member 210 includes an insulating member 216 disposed within the longitudinal slot thereof, while jaw member 220 includes an electrical cutting assembly 225 disposed within the longitudinal slot thereof. More specifically, insulating member 216 of jaw member 210 has a longitudinally-extending cut-out 217 defined by a base surface 217a and a pair of angled side surfaces 217b. Cutting electrode 228 of electrical cutting assembly 225 of jaw member 220 extends beyond tissue-contacting surface 222 of jaw member 220 towards jaw member 210 and is configured for receipt within cut-out 217 of insulating member 216 of jaw member 210 when jaw members 210, 220 are moved to the approximated position, as shown in FIG. 4. Further, cutting electrode 228 functions as a gap stop for defining a minimum gap distance between tissue-contacting surfaces 212, 222 of jaw members 210, 220, respectively, e.g., the minimum gap distance is defined when cutting electrode 228 abuts base surface 217a of insulating member 216.

Continuing with reference to FIG. 4, angled side surfaces 217b of cut-out 217 are configured to guide cutting electrode 228 into cut-out 217 to align jaw members 210, 220 with one another in the event jaws members 210, 220 are splayed/misaligned with one another during approximation. That is, upon contact of cutting electrode 228 with either of angled surfaces 217b of cut-out 217 during approximation of jaw members 210, 220, the inwardly-facing angled surfaces 217b urge cutting electrode 228 inwardly towards a center of insulating member 216, thereby urging jaw member 220 into alignment with jaw member 210. Ensuring alignment of jaw members 210, 220 and, more particular, cutting electrode 228 and insulating member 216, helps maintain sufficient and substantially equal spacing between cutting electrode 228 and tissue-contacting surface 212 on either side of cutting electrode 228 so as to reduce current concentrations and provide a more uniform distribution of current flow from cutting electrode 228, through tissue, to tissue-contacting surface 212 (and/or tissue-contacting surface 222). As a result, effective energy-based tissue cutting can be more readily achieved and damage to surrounding tissue can be minimized. Further, the alignment of jaw members 210, 220 as described above not only facilitates electrical cutting, but also facilitates the formation of an effective tissue seal and minimizes damage to surrounding tissue during conduction of energy between tissue-contacting surfaces 212, 222 to treat, e.g., seal, tissue, as alignment between tissue-contacting surfaces 212, 222 is also achieved via the alignment of jaw members 210, 220.

The width of cut-out 217 of insulating member 216 and, more particularly, the width of base surface 217a thereof, may be varied depending on the precision of alignment desired. That is, if more precise alignment is desired, base surface 217a may define a relatively narrow width that approaches the width of cutting electrode 228 such that angled surfaces 217b urge jaw member 220 into more precise alignment with jaw member 210. On the other hand, if it is only desired to align jaw members 210, 220 to within an acceptable range, base surface 217a may define a larger width such that angled surfaces 217b urge cutting electrode 228 only so much as required to maintain jaw members 210, 220 within the acceptable alignment range. Further, although described above with respect to the static cutting configuration of end effector assembly 200, the above-described configuration may also be employed for dynamic-cutting configurations, e.g., wherein the jaw members 110, 120 (FIG. 3A) are urged into alignment upon translation of the knife blade (not shown) therethrough.

Turning now to FIGS. 5A-5D, various jaw members 310, 410, 510, 610 configured for use in conjunction with jaw member 220 (FIGS. 3B and 4), or any other suitable jaw member including a centrally disposed and longitudinally-extending electrical cutting assembly are provided in accordance with the present disclosure. Each of jaw members 310, 410, 510, 610 will be described in detail, in turn, below. The features and aspects of any of jaw members 310, 410, 510, 610 may apply similarly to or may be used in conjunction with the features and aspects of any or all of the other jaw members 310, 410, 510, 610.

As shown in FIG. 5A, in conjunction with FIGS. 3B and 4, jaw member 310 generally includes an outer jaw housing 311, an electrically-conductive tissue-contacting surface 312 positioned on outer jaw housing 311 and configured to oppose the tissue-contacting surface 222 of the other jaw member, e.g., jaw member 220, and a proximal flange 314 for pivotably coupling jaw member 310 to shaft 12 (FIG. 1) and jaw member 220. Tissue-contacting surface 312 defines a longitudinal slot 315 extending therealong that includes an insulating member 316 disposed therein. Similarly as described above with respect to end effector assembly 200, insulating member 316 of jaw member 310 is configured to oppose cutting electrode 228 of electrical cutting assembly 225 of jaw member 220 when jaw members 310, 220 are moved to the approximated position. As can be appreciated, if jaw members 310, 220 becomes splayed/misaligned relative to one another during approximation, cutting electrode 228 is no longer centered relative to insulating member 316 but, rather, is closer to tissue-contacting surface 312 on one side thereof and further from tissue-contacting surface 312 on the other side thereof. With cutting electrode 228 unevenly positioned relative to tissue-contacting surface 312, current concentrations are established between cutting electrode 228 and the closer side of tissue-contacting surface 312 as compared to the further-away side of tissue-contacting surface 312, potentially compromising the effectiveness of the electrical tissue cut and/or damaging surrounding tissue.

In order to account for such splaying/misalignment, longitudinal slot 315 and insulating member 316 each define flared configurations that gradually widen from the proximal ends 315a, 316a to the distal ends 315b, 316b, respectively, thereof. That is, since the offset distance resulting from splaying/misalignment of jaw members 310, 220 generally increases as the distance from the pivot point increases, jaw member 310 defines a configuration wherein the widths of longitudinal slot 315 and insulating member 316 generally increase as the distance from the pivot point, e.g., proximal flange 314, increases. As such, cutting electrode 228 is inhibited from being positioned in close approximation with tissue-contacting surface 212 on either side of cutting electrode 228 and, thus, current concentrations as a result of splaying/misalignment of jaw members 310, 220 are avoided.

Turning now to FIG. 5B, in conjunction with FIGS. 3B and 4, another embodiment of a jaw member 410, similar to jaw member 310 (FIG. 5A), generally includes an outer jaw housing 411, an electrically-conductive tissue-contacting surface 412 positioned on outer jaw housing 411 and configured to oppose the tissue-contacting surface 222 of the other jaw member, e.g., jaw member 220, and a proximal flange for pivotably coupling jaw member 410 to shaft 12 (FIG. 1) and jaw member 220. Tissue-contacting surface 412 defines a longitudinal slot 415 having an insulating member 416 disposed therein that is configured to oppose cutting electrode 228 of electrical cutting assembly 225 of jaw member 220 when jaw members 410, 220 are moved to the approximated position. Longitudinal slot 415 and insulting member 416 each include an expanded distal portion 417, 418, respectively, that is configured to receive the distal end of cutting electrode 228 of electrical cutting assembly 225 therein. As shown, expanded distal portions 417, 418 of longitudinal slot 415 and insulating member 416, respectively, define generally oval-shaped configurations, although other configurations are also contemplated. Expanded distal portions 417, 418 provide increased spacing between cutting electrode 228 and tissue-contacting surface 412 of jaw member 410 at the distal end of cutting electrode 228 when jaw members 410, 220 are disposed in the approximated position. This increased spacing between the distal end of cutting electrode 228 and tissue-contacting surface 412 of jaw member 410 helps reduce current concentrations at the distal end of cutting electrode 228 and more evenly distribute current along cutting electrode 228. Such a feature is particularly advantageous in that current concentrations typically occur at the distal end of cutting electrode 228 due to the fact that current may flow out of the distal end, sides, and top of cutting electrode 228 at the distal end thereof, as compared to intermediate portions of cutting electrode 228, wherein current may only flow out from the sides and top of cutting electrode 228. The above-described configuration may also be utilized in conjunction with an energized, translatable knife blade (not shown), such as that described above with respect to FIG. 3A.

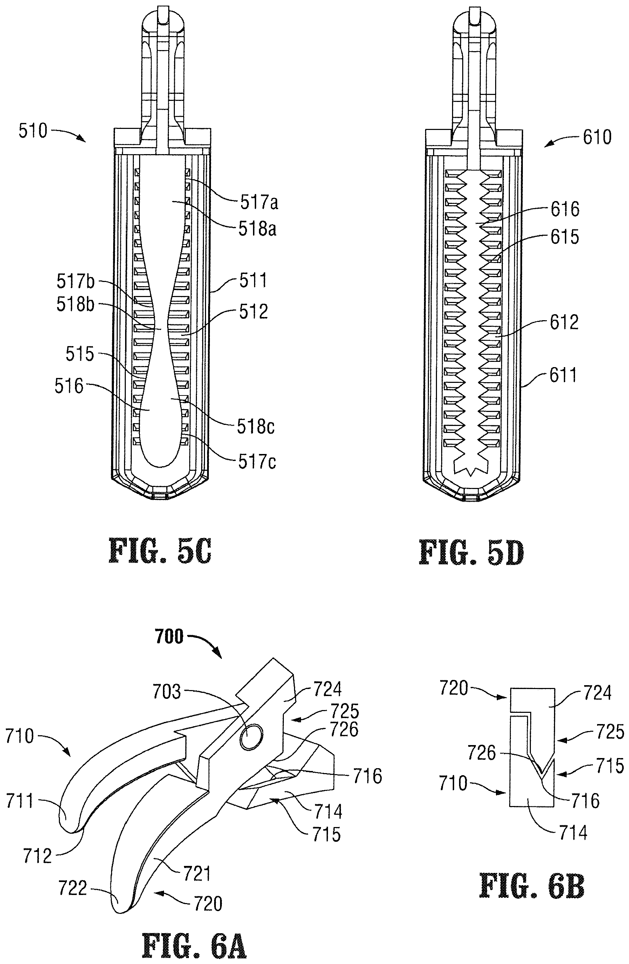

As shown in FIG. 5C, in conjunction with FIGS. 3B and 4, another embodiment of a jaw member 510, similar to jaw members 310, 410 (FIGS. 5A and 5B, respectively), generally includes an outer jaw housing 511 and an electrically-conductive tissue-contacting surface 512 positioned on outer jaw housing 511 and configured to oppose the tissue-contacting surface 222 of the other jaw member, e.g., jaw member 220. Tissue-contacting surface 512 defines a longitudinal slot 515 having an insulating member 516 disposed therein that is configured to oppose cutting electrode 228 of electrical cutting assembly 225 of jaw member 220 when jaw members 510, 220 are moved to the approximated position. Longitudinal slot 515 and insulting member 516 each include a proximal portion 517a, 518a, a central portion 517b, 518b, and a distal portion 517c, 518c, respectively. Central portions 517b, 518b of longitudinal slot 515 and insulating member 516, respectively, define narrowed, inwardly-bowed configurations such that, upon approximation of jaw members 510, 220, cutting electrode 228 is disposed in close proximity to tissue-contacting surface 512 adjacent the central portion of jaw member 510, but is further-spaced from tissue-contacting surface 512 adjacent the proximal and distal portions of jaw member 510. This configuration establishes current concentrations adjacent the central portion of jaw member 510 upon grasping of tissue between jaw members 510, 220 and activating cutting electrode 228. As such, electrical cutting of tissue is initiated towards the center of tissue (which is grasped adjacent the central portion of jaw members 510, 220) as opposed to the edges of tissue (which are disposed adjacent the proximal and distal portions of jaw members 510, 220), thus facilitating a complete and effective tissue cut. The above-described configuration may also be utilized in conjunction with an energized, translatable knife blade (not shown), such as that described above with respect to FIG. 3A.

Turning now to FIG. 5D, in conjunction with FIGS. 3B and 4, another embodiment of a jaw member 610, similar to jaw members 310, 410 (FIGS. 5A and 5B, respectively), generally includes an outer jaw housing 611 and an electrically-conductive tissue-contacting surface 612 positioned on outer jaw housing 611 and configured to oppose the tissue-contacting surface 222 of the other jaw member, e.g., jaw member 220. Tissue-contacting surface 612 defines a longitudinal slot 615 having an insulating member 616 disposed therein that is configured to oppose cutting electrode 228 of electrical cutting assembly 225 of jaw member 220 when jaw members 610, 220 are moved to the approximated position. Longitudinal slot 615 and insulting member 616 define irregular peripheral edges, e.g., zigzagged peripheral edges (as shown), although other configurations are also contemplated. As a result of this configuration, tissue-contacting surface 612 of jaw member 610 likewise defines a zigzagged inner edge, e.g., at the interface between tissue-contacting surface 612 and longitudinal slot 615 and insulating member 616. Such a configuration helps distribute the current from cutting electrode 228, thus helping to alleviate current concentrations, e.g., in the event of splaying/misalignment of jaw members 610, 220 or otherwise. The above-described configuration may also be utilized in conjunction with an energized, translatable knife blade (not shown), such as that described above with respect to FIG. 3A. Further, as an alternative to or in addition to opposing jaw member 220, any of the above-described configurations of insulating members (see FIGS. 5A-5D) may be incorporated into the insulating member that surrounds electrical cutting member 228, e.g., insulating member 226.

FIGS. 6A-6B show another embodiment of an end effector assembly 700 provided in accordance with the present disclosure. End effector assembly 700, as will be described in greater detail below, is configured to inhibit jaw splaying/misalignment, thereby facilitating the grasping, sealing, and/or cutting (statically or dynamically) of tissue.

As best shown in FIG 6A, end effector assembly 700, similar to end effector assemblies 100, 200 (FIGS. 3A and 3B, respectively), includes first and second jaw members 710, 720, each including an outer insulative jaw housing 711, 721 and an electrically-conductive tissue-contacting surface 712, 722, respectively. Tissue-contacting surfaces 712, 722 are adapted to electrically couple to a source of energy (not shown) such that energy may be selectively supplied to tissue-contacting surface 712 and/or tissue-contacting surface 722 and conducted therebetween and through tissue disposed between jaw members 710, 720 to treat, e.g., seal, tissue. Either or both jaw members 710, 720 may further include a longitudinally-extending knife channel (not shown) to facilitate reciprocation of a mechanical or energizable knife blade (not shown) between jaw members 710, 720, or may be configured for static cutting, e.g., wherein one of the jaw members 710, 720 includes a cutting electrode (not shown) and the other jaw member 710, 720 includes an opposed insulating member (not shown).

With continued reference to FIGS. 6A-6B, each jaw member 710, 720 further includes a proximal flange 714, 724 extending proximally therefrom. A pivot member 703 pivotably couples proximal flanges 714, 724 to one another, thus allowing jaw members 710, 720 to pivot relative to one another between spaced-apart and approximated positions for grasping tissue therebetween. One of the proximal flanges, e.g., proximal flange 724 of jaw member 720, includes an engagement portion 725 defining a protrusion 726 having a triangular-shaped cross-sectional configuration, although other configurations are also contemplated. The other proximal flange, e.g., proximal flange 714 of jaw member 710, includes an engagement portion 715 defining a recess 716 having a triangular-shaped cross-sectional configuration, although any other suitable complementary configurations of engagement portions 715, 725 are also contemplated.

During use, as jaw members 710, 720 are approximated relative to one another, protrusion 726 is received within recess 716 and is centered relative thereto, e.g., as a result of the complementary triangular-shaped configurations of engagement portions 715, 725, such that jaw members 710, 720 are maintained in alignment with one another and jaw splaying is inhibited. As discussed above, jaw alignment facilitates proper grasping of tissue, treating, e.g., sealing, of tissue, and cutting of tissue (either mechanically, electromechanically, or electrically).

When protrusion 726 is received within recess 716, the abutment of engagement potions 715, 725 inhibits further approximation of jaw members 710, 720 relative to one another. Thus, engagement portions 715, 725 can also be configured to define the minimum gap distance between jaw members 710, 720 when disposed in the approximated position.

Complementary engagement portions 715, 725 of jaw members 710, 720 may also be utilized to facilitate alignment of jaw members 710, 720 and setting of the minimum gap distance during manufacturing. More specifically, during manufacturing, and prior to pivotably coupling proximal flanges 714, 724 of jaw members 710, 720, respectively, to one another, jaw members 710, 720 may first be moved to the approximated position with the desired minimum gap distance therebetween such that protrusion 726 is received within recess 716, thereby aligning jaw members 710, 720 relative to one another. With jaw members 710, 720 maintained in this aligned position and defining the minimum gap distance, holes for receipt of pivot member 703 may be drilled (or otherwise formed) through flanges 714, 724 of jaw members 710, 720, respectively. Thus, when end effector assembly 700 is assembled, e.g., once pivot member 703 is engaged within the holes in flanges 714, 724 to pivotably couple jaw members 710, 720 to one another, proper jaw alignment is achieved upon movement of jaw members 710, 720 to the approximated position and the desired minimum gap distance is defined between jaw members 710, 710 upon receipt of protrusion 726 within recess 716.

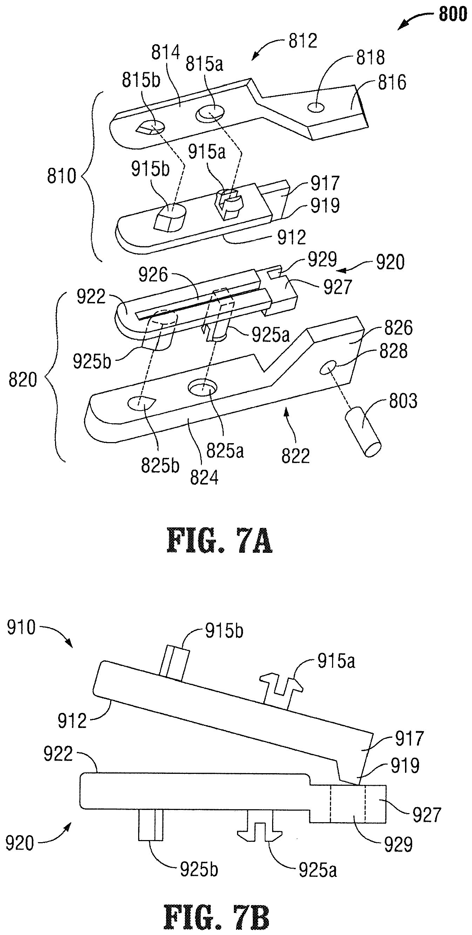

Turning now to FIGS. 7A-7B, another embodiment of an end effector assembly 800 provided in accordance with the present disclosure is described. End effector assembly 800 includes first and second jaw members 810, 820, each of which includes a fixed jaw frame 812, 822, respectively, and a replaceable component 910, 920, respectively, that is selectively engagable with the respective jaw frame 812, 822 to form the fully assembled jaw members 810, 820, respectively. Jaw frames 812, 822 each include a base portion 814, 824 having first and second engagement apertures 815a, 815b and 825a, 825b, respectively, and a proximal flange 816, 826 having a pivot aperture 818, 828, respectively, configured to receive pivot member 803 for pivotably coupling jaw members 810, 820 to one another.

Replaceable components 910, 920 of jaw members 810, 820, respectively, define the tissue-contacting surfaces 912, 922 of jaw members 810, 820, respectively, and are adapted to connect to a source of energy (not shown) for conducting energy therebetween and through tissue grasped between jaw members 810, 820 to treat, e.g., seal tissue. One or both replaceable components 910, 920, e.g., replaceable component 920, may include a longitudinally-extending knife channel 926 to facilitate reciprocation of a mechanical or energizable knife blade (not shown) between jaw members 810, 820. Alternatively, end effector assembly 800 may be configured for static cutting, e.g., wherein one of the jaw members 810, 820 includes a cutting electrode (not shown) and the other jaw member 810, 820 includes an opposed insulating member (not shown). Each replaceable component 910, 920 further includes a pair of engagement members 915a, 915b and 925a, 925b configured for receipt within respective first and second engagement apertures 815a, 815b and 825a, 825b, respectively, of jaw frames 812, 822. More specifically, engagement members 915a, 925a each include a pair of outwardly-biased legs configured for snap-fit engagement within engagement apertures 815a, 825a, respectively (although other engagement configurations are also contemplated). Engagement members 915b, 925b and engagement apertures 815b, 825b, respectively, on the other hand, define complementary, non-circular configurations such that, upon receipt of engagement members 915b, 925b within engagement apertures 815b, 825b, and with engagement members 915a, 925a snap-fittingly engaged within engagement apertures 815a, 825a, substantial movement of replaceable components 910, 920 relative to respective jaw frames 812, 822 is inhibited.

Each replaceable component 910, 920 further includes a proximal flange 917, 927, respectively. One of the proximal flanges 917, 927, e.g., proximal flange 917 of replaceable component 910, defines a tab 919 extending therefrom, while the other proximal flange 917, 927, e.g., proximal flange 927 of replaceable component 920, defines a slot 929 configured to receive tab 919. In embodiments, e.g., in embodiments where a reciprocating mechanical knife blade (not shown) is provided, flanges 917, 927 may be offset from the respective centers of replaceable components 910, 920 so as to avoid interfering with reciprocation of the knife blade (not shown).

During use, as jaw members 810, 820 are approximated relative to one another, tab 919 of proximal flange 917 is received within slot 929 of proximal flange 927 (see FIG. 7B) such that jaw members 810, 920 are maintained in alignment with one another and jaw splaying is inhibited. Thus, the above-described configuration of end effector assembly 800 provides for engagement members 915a, 915b, 925a, 925b and corresponding engagement apertures 815a, 815b, 825a, 825b that inhibit substantial movement of replaceable components 910, 920 relative to respective jaw frames 812, 822, and flanges 917, 927 that inhibit substantial movement of replaceable components 910, 920 relative to one another. Accordingly, proper jaw alignment can be readily achieved, thereby facilitating the grasping, treating, e.g., sealing, and/or cutting of tissue.

From the foregoing and with reference to the various figure drawings, those skilled in the art will appreciate that certain modifications can also be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope of the claims appended hereto.

## Claims

1. A forceps, comprising:
an end effector assembly (200) including first and second jaw members (220, 210), at least one of the jaw members (220, 210) movable relative to the other between a spaced-apart position and an approximated position for grasping tissue therebetween, each jaw member (220, 210) including an electrically-conductive tissue-contacting surface (222, 212) adapted to connect to a source of energy to treat tissue grasped between the jaw members (220, 210), the first jaw member (220) including a cutting electrode (228) adapted to connect to the source of energy to cut tissue grasped between the jaw members (220, 210), the second jaw member (210) including a first insulative member (216) positioned to oppose the cutting electrode (228), the first insulative member (216) configured to guide the cutting electrode (228) into alignment with the first insulative member (216) upon approximation of the jaw members (220, 210) to thereby align the jaw members (220, 210) relative to one another upon approximation of the jaw members (220, 210),
wherein the first insulative member (216) defines a cut-out formed from at least one angled surface (217b), the at least one angled surface configured to guide the cutting electrode (228) into alignment within the cut-out upon approximation of the jaw members (220, 210),
**characterised in that** the cut-out is defined by a base surface (217a) of the first insulative member (216) and a pair of angled surfaces (217b) of the first insulative member (216) disposed on either side of the base surface (217a), the angled surfaces (217b) configured to guide the cutting electrode (228) into alignment with the base surface (217a), wherein, in the approximated position of the jaw members (220, 210), the cutting electrode (228) contacts the base surface (217a) of the first insulative member (216) to define a minimum gap distance between the first and second jaw members (220, 210).

2. The forceps according to claim 1, further comprising a second insulative member (226) surrounding the cutting electrode (228) and configured to electrically insulate the cutting electrode (228) and tissue-contacting surface (222) of the first jaw member (220) from one another.

3. The forceps according to any preceding claim, wherein the tissue-contacting surfaces (222, 212) of the jaw members (220, 210) are configured to conduct energy therebetween and through tissue grasped between the jaw members (220, 210) to treat tissue.

4. The forceps according to any preceding claim, wherein the cutting electrode (228) is configured to conduct energy to at least one of the tissue-contacting surfaces (222, 212) and through tissue grasped between the jaw members to cut tissue.

## Patentansprüche

1. Zange, umfassend:
eine Endeffektoranordnung (200), die erste und zweite Backenelemente (220, 210) aufweist, wobei mindestens eines der Backenelemente (220, 210) in Bezug auf das andere zwischen einer beabstandeten Position und einer angenäherten Position zum Ergreifen von Gewebe dazwischen beweglich ist, wobei jedes Backenelement (220, 210) eine elektrisch leitende Gewebekontaktfläche (222, 212) aufweist, die zum Verbinden mit einer Stromversorgung zum Behandeln von Gewebe ausgelegt ist, das zwischen den Backenelementen (220, 210) ergriffen wird, wobei das erste Backenelement (220) eine Schneidelektrode (228) aufweist, die zum Verbinden der Stromversorgung zum Schneiden von Gewebe, das zwischen den Backenelementen (220, 210) ergriffen wurde, ausgelegt ist, wobei das zweite Backenelement (210) ein erstes isolierendes Element (216) aufweist, das zum Gegenüberliegen der Schneidelektrode (228) positioniert ist, wobei das erste isolierende Element (216) zum Führen der Schneidelektrode (228) in Ausrichtung mit dem ersten isolierenden Element (216) nach Annäherung der Backenelemente (220, 210) ausgelegt ist, um so die Backenelemente (220, 210) in Bezug aufeinander nach Annäherung der Backenelemente (220, 210) auszurichten,
wobei das erste isolierende Element (216) einen Ausschnitt definiert, der aus mindestens einer Abwinklungsfläche (217b) gebildet ist, wobei die mindestens eine Abwinklungsfläche zum Führen der Schneidelektrode (228) in Ausrichtung innerhalb des Ausschnitts nach Annäherung der Backenelemente (220, 210) konfiguriert ist,
**dadurch gekennzeichnet, dass** der Ausschnitt durch eine Basisfläche (217a) des ersten isolierenden Elements (216) und ein Paar abgewinkelter Flächen (217b) des ersten isolierenden Elements (216) definiert ist, die auf jeder Seite der Basisfläche (217a) positioniert sind, wobei die abgewinkelten Flächen (217b) zum Führen der Schneidelektrode (228) in Ausrichtung mit der Basisfläche (217a) konfiguriert sind, wobei in der angenäherten Position der Backenelemente (220, 210) die Schneidelektrode (228) mit der Basisfläche (217a) des ersten isolierenden Elements (216) in Kontakt tritt, um einen minimalen Spaltabstand zwischen dem ersten und zweiten Backenelement (220, 210) zu definieren.

2. Zange nach Anspruch 1, weiter umfassend ein zweites isolierendes Element (226), das die Schneideelektrode (228) umgibt und zum elektrischen Isolieren der Schneideelektrode (228) und der gewebekontaktierenden Fläche (222) des ersten Backenelements (220) voreinander konfiguriert ist.

3. Zange nach einem der vorstehenden Ansprüche, wobei die gewebekontaktierenden Flächen (222, 212) der Backenelemente (220, 210) zum Leiten von Strom dazwischen und durch das Gewebe konfiguriert sind, das zwischen den Backenelementen (220, 210) zum Behandeln von Gewebe ergriffen wird.

4. Zange nach einem der vorstehenden Ansprüche, wobei die Schneidelektrode (228) zum Leiten von Strom zu mindestens einer der gewebekontaktierenden Flächen (222, 212) und durch Gewebe konfiguriert ist, das zwischen den Backenelementen zum Schneiden von Gewebe ergriffen ist.

## Revendications

1. Pince, comprenant :
un ensemble effecteur d'extrémité (200) incluant des premier et second éléments de mors (220, 210), au moins un des éléments de mors (220, 210) étant mobile par rapport à l'autre entre une position espacée et une position rapprochée pour saisir du tissu entre ces derniers, chaque élément de mors (220, 210) incluant une surface électriquement conductrice entrant en contact avec du tissu (222, 212), conçue pour se connecter à une source d'énergie pour traiter le tissu saisi entre les éléments de mors (220, 210), le premier élément de mors (220) incluant une électrode de coupe (228) conçue pour connexion à la source d'énergie pour couper du tissu saisi entre les éléments de mors (220, 210), le second élément de mors (210) incluant un premier élément isolant (216) positionné pour faire face à l'électrode de coupe (228), le premier élément isolant (216) étant configuré pour guider l'électrode de coupe (228) dans l'alignement avec le premier élément isolant (216) lors du rapprochement des éléments de mors (220, 210) pour aligner de ce fait les éléments de mors (220, 210) l'un par rapport à l'autre lors du rapprochement des éléments de mors (220, 210),
dans laquelle le premier élément isolant (216) définit une découpe formée à partir d'au moins une surface inclinée (217b), l'au moins une surface inclinée étant configurée pour guider l'électrode de coupe (228) dans l'alignement à l'intérieur de la découpe lors du rapprochement des éléments de mors (220, 210),
**caractérisée en ce que** la découpe est définie par une surface de base (217a) du premier élément isolant (216) et un couple de surfaces inclinées (217b) du premier élément isolant (216) disposées de chaque côté de la surface de base (217a), les surfaces inclinées (217b) étant configurées pour guider l'électrode de coupe (228) dans l'alignement avec la surface de base (217a), dans laquelle, dans la position rapprochée des éléments de mors (220, 210), l'électrode de coupe (228) entre en contact avec la surface de base (217a) du premier élément isolant (216) pour définir une distance d'espacement minimale entre les premier et second éléments de mors (220, 210).

2. Pince selon la revendication 1, comprenant en outre un second élément isolant (226) entourant l'électrode de coupe (228) et configuré pour électriquement isoler l'une de l'autre l'électrode de coupe (228) et la surface entrant en contact avec du tissu (222) du premier élément formant mâchoire (220).

3. Pince selon l'une quelconque des revendications précédentes, dans laquelle les surfaces entrant en contact avec du tissu (222, 212) des éléments de mors (220, 210) sont configurées pour conduire l'énergie entre ces dernières et à travers le tissu saisi entre les éléments de mors (220, 210) pour traiter le tissu.

4. Pince selon l'une quelconque des revendications précédentes, dans laquelle l'électrode de coupe (228) est configurée pour conduire l'énergie jusqu'à au moins une des surfaces entrant en contact avec du tissu (222, 212) et à travers le tissu saisi entre les éléments de mors pour couper le tissu.
